(19) Europäisches Patentamt
European Patent Office
Office européen des brevets

(11) EP 4 614 382 A1

(12) EUROPEAN PATENT APPLICATION

(43) Date of publication:
10.09.2025 Bulletin 2025/37

(21) Application number: 25161977.1

(22) Date of filing: 06.03.2025

(51) International Patent Classification (IPC):
*G06F 30/27* (2020.01)     *G06N 20/00* (2019.01)

(52) Cooperative Patent Classification (CPC):
G06N 20/00; G06N 3/006; G16C 60/00;
G16C 20/30; G16C 20/70

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 07.03.2024 IN 202421016615

(71) Applicant: Tata Consultancy Services Limited
Maharashtra (IN)

(72) Inventors:
• MUHAMMED, Bilal
695581 Kerala (IN)

• BHATTACHARJEE, Akash
411028 Pune, Maharashtra (IN)
• BASAVARSU, Purushottham Gautham
411057 Pune, Maharashtra (IN)
• TENNYSON, Gerald
411057 Pune, Maharashtra (IN)
• JOSHI, Amol Dilip
411057 Pune, Maharashtra (IN)

(74) Representative: Goddar, Heinz J.
Boehmert & Boehmert
Anwaltspartnerschaft mbB
Pettenkoferstrasse 22
80336 München (DE)

(54) **METHODS AND SYSTEMS FOR AUTOMATED DESIGN OF MATERIALS AND ITS MANUFACTURING PROCESS FOR DESIRED PROPERTIES**

(57) The disclosure relates generally to methods and systems for automated design of materials and the manufacturing process for desired properties. Conventional automated materials design techniques do not perform an integrated design of (i) a material composition and (ii) their manufacturing processing steps. The present disclosure addresses this gap by using a multi-agent setup for automated design, wherein a distinct Reinforcement learning (RL) agent is used to mirror the composition selection (CS) and various sequential manufacturing process steps (PS) involved in its manufacturing route. The distinct RL agents learn from both past design data and computational models (empirical/analytical/physics-based models) representing the design process. The present disclosure also integrates other important parameters such as manufacturability, ESG norms, cost, process energy etc. and their relative importance into the design decision making process of the RL agents by expressing them as reward components upon which the RL agents are trained.

FIG. 2

EP 4 614 382 A1

**Description**

CROSS-REFERENCE TO RELATED APPLICATIONS AND PRIORITY

The present application claims priority to Indian application no. 202421016615, filed on March 07, 2024.

TECHNICAL FIELD

The disclosure herein generally relates to the field of material design, and more specifically to methods and systems for automated design of materials and its manufacturing process for desired properties.

BACKGROUND

Material design and development is a complex, costly, and time-consuming process with a high resource requirement. Materials are designed for a specific application which requires a set of target properties. The final properties of the materials are determined by the composition and the structure obtained via the processing of the material through a manufacturing route. There are multiple manufacturing process routes available in the art to develop the material. Each manufacturing process route often involves multiple sequential manufacturing process steps. For example, a hot rolled steel sheet is produced through two different manufacturing process routes viz., a conventional casting route or a thin slab casting route depending on the final property requirements. Along with the target properties of the material, other parameters such as a cost, a manufacturability, constraints arriving from environment, social, & governance (ESG) norms, production capabilities, and so on are taken into consideration during the production to meet optimal design.

Conventional techniques for the materials design involve extensive experimental trials and utilizing experience of the designers, and processing-structure-property relations known. Most of the conventional automated materials design techniques either focus on correlating between composition of the materials and their properties or uses a process model to corelate between the process parameters and the properties of the material at a single process level but does not take into consideration the entire process chain for manufacturing of the materials that determine the underlying structure evolution and thereby the properties of the developed material. The conventional automated materials design techniques lack an integrated design of (i) a material composition and (ii) their manufacturing processing steps.

SUMMARY

Embodiments of the present disclosure present technological improvements as solutions to one or more of the above-mentioned technical problems recognized by the inventors in conventional systems.

In an aspect, a processor-implemented method for automated design of materials and its manufacturing process for desired properties is provided. The method including the steps of: receiving a historical design data associated to a plurality of materials, from a repository; creating a training dataset of each material of the plurality of materials, from the historical design data, to obtain a plurality of training data sets associated with the plurality of materials, wherein the training data set of each of the plurality of materials comprises (i) an achieved value of each of one or more properties of the material, (ii) a value of each of one or more composition elements present in the material, (iii) a value of each of one or more process parameters of each of one or more sequential manufacturing process steps present in each of one or more manufacturing process routes that produces the material; receiving a target value of each of the one or more properties of each material of the plurality of materials; training a set of reinforcement learning (RL) models, using the plurality of training data sets and the target value of each of the one or more properties of each material of the plurality of materials to obtain a set of trained RL models, wherein each RL model in the set of RL models is defined for (i) a composition selection from the one or more composition elements, and (ii) each of the one or more sequential manufacturing process steps present in each of the one or more manufacturing process routes; receiving the target value of each of the one or more properties of a desired material; and passing the target value of each of the one or more properties of the desired material, to the set of trained RL models, to sequentially predict (i) one or more material compositions, and (ii) the value of each of the one or more process parameters of each of the one or more sequential manufacturing process steps present in each of the one or more manufacturing process routes, for each of the one or more material compositions, and wherein each of the one or more material compositions comprises the value of each of the one or more composition elements.

In another aspect, a system for automated design of materials and its manufacturing process for desired properties is provided. The system includes: a memory storing instructions; one or more Input/Output (I/O) interfaces; and one or more hardware processors coupled to the memory via the one or more I/O interfaces, wherein the one or more hardware processors are configured by the instructions to: receive a historical design data associated to a plurality of materials, from a repository; create a training dataset of each material of the plurality of materials, from the historical design data, to obtain a plurality of training data sets associated with the plurality of materials, wherein the training data set of each of the plurality of materials comprises (i) an achieved value of each of one or more properties of the material, (ii) a value of each of one or more composition elements present in the material, (iii) a value of each of one or more process parameters of each of one or more sequential manufacturing process steps present in each of one or more manufacturing process routes that produces the material; receive a target value of each of the one or more properties of each material of the plurality of materials; train a set of reinforcement learning (RL) models, using the plurality of training data sets and the target value of each of the one or more properties of each material of the plurality of materials to obtain a set of trained RL models, wherein each RL model in the set of RL models is defined for (i) a composition selection from the one or more composition elements, and (ii) each of the one or more sequential manufacturing process steps present in each of the one or more manufacturing process routes; receive the target value of each of the one or more properties of a desired material; and pass the target value of each of the one or more properties of the desired material, to the set of trained RL models, to sequentially predict (i) one or more material compositions, and (ii) the value of each of the one or more process parameters of each of the one or more sequential manufacturing process steps present in each of the one or more manufacturing process routes, for each of the one or more material compositions, and wherein each of the one or more material compositions comprises the value of each of the one or more composition elements.

In yet another aspect, there are provided one or more non-transitory machine-readable information storage mediums comprising one or more instructions which when executed by one or more hardware processors cause: receiving a historical design data associated to a plurality of materials, from a repository; creating a training dataset of each material of the plurality of materials, from the historical design data, to obtain a plurality of training data sets associated with the plurality of materials, wherein the training data set of each material of the plurality of materials comprises (i) an achieved value of each of one or more properties of the material, (ii) a value of each of one or more composition elements present in the material, (iii) a value of each of one or more process parameters of each of one or more sequential manufacturing process steps present in each of one or more manufacturing process routes that produces the material; receive a target value of each of the one or more properties of each material of the plurality of materials; training a set of reinforcement learning (RL) models, using the plurality of training data sets and the target value of each of the one or more properties of each material of the plurality of materials to obtain a set of trained RL models, wherein each RL model in the set of RL models is defined for (i) a composition selection from the one or more composition elements, and (ii) each of the one or more sequential manufacturing process steps present in each of the one or more manufacturing process routes; receiving the target value of each of the one or more properties of a desired material; and passing the target value of each of the one or more properties of the desired material, to the set of trained RL models, to sequentially predict (i) one or more material compositions, and (ii) the value of each of the one or more process parameters of each of the one or more sequential manufacturing process steps present in each of the one or more manufacturing process routes, for each of the one or more material compositions, and wherein each of the one or more material compositions comprises the value of each of the one or more composition elements.

In an embodiment, wherein training the set of RL models, using the plurality of training data sets to obtain the set of trained RL models, comprises: defining a global reward function of the set of RL models, as a weighted function of the one or more properties of the material, and one or more characteristics of the material; defining a local reward function of each RL model in the set of RL models, as a weighted function of one or more of: (i) one or more sequential manufacturing process step specific constraints, (ii) one or more evaluation metrics, (iii) one or more material structure state constraints, and (iv) the one or more characteristics of the material that are dependent of each sequential manufacturing process step, and wherein the one or more evaluation metrics comprises one or more environment, social, and governance (ESG) norms, one or more economic indices, and one or more manufacturability indices; defining a training environment of each RL model in the set of RL models, using the local reward function of associated RL model, and by utilizing a set of computational simulation models, wherein the training environment of each RL model determines a next state, the value of the local reward function, and a learning episode completion status, for a given state, based on an action taken by the corresponding RL model; transforming each training data set of the plurality of training data sets along with the target value of each of the one or more properties of each material of the plurality of materials, at a time, to determine (i) a state, (ii) the next state, and (iii) the action, (iv) the reward, and (v) the learning episode completion status, of each RL model in the set of RL models; and iteratively training each RL model in the set of RL models, defined for (i) the composition selection, and (ii) each of the one or more sequential manufacturing process steps present in each of the one or more manufacturing process routes, in a reverse sequential order, by utilizing the training environment of the associated RL model and each training dataset present in the plurality of training data sets, to obtain the set of trained RL models.

In an embodiment, the state of each RL model of the composition selection, and each sequential manufacturing process step, is defined with respect to one or more of (i) the value of each of the one or more process parameters of each of the one or more sequential manufacturing process steps that are precedent to the corresponding sequential manufacturing process step present in each manufacturing process route, (ii) the value of each of the one or more composition elements, (iii) the target value of each of one or more properties of the material, (iv) the one or more evaluation metrics, and (v) one or more material structure state parameters.

In an embodiment, the action of each RL model of the composition selection, and each sequential manufacturing process step, is defined with respect to the value of each of one or more composition elements present in the material, the value of each of the one or more process parameters of the corresponding sequential manufacturing process step present in each manufacturing process route, respectively.

In an embodiment, the reward of each RL model is defined as a sum of a value of the local reward function of the associated RL model and the value of the global reward function.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is an exemplary block diagram of a system for automated design of materials and its manufacturing process for desired properties, in accordance with some embodiments of the present disclosure.

FIG. 2 is an exemplary block diagram illustrating a plurality of modules of the system of FIG. 1, for automated design of materials and its manufacturing process for desired properties, in accordance with some embodiments of the present disclosure.

FIGS. 3A-3B illustrate exemplary flow diagrams of a processor-implemented method for automated design of materials and its manufacturing process for desired properties, using the system of FIG. 1, in accordance with some embodiments of the present disclosure.

FIG. 4 is a flowchart comprising steps for creating a plurality of training datasets of a plurality of materials, from the historical design data, in accordance with some embodiments of the present disclosure.

FIG. 5A shows an exemplary design framework for automated design of materials and manufacturing process designs for targeted requirements, in accordance with some embodiments of the present disclosure.

FIG. 5B shows exemplary conventional manufacturing process routes for producing a hot rolled steel sheet.

FIG. 6 shows an exemplary setup of the RL problem for a material design decision making problem, in accordance with some embodiments of the present disclosure.

FIG. 7 is a flowchart comprising steps for training the set of RL models, using the plurality of training data sets to obtain the set of trained RL models, in accordance with some embodiments of the present disclosure.

FIG. 8 is a flowchart comprising steps for normalizing and assigning relative weights to reward components, in accordance with some embodiments of the present disclosure.

FIG. 9 is a flowchart comprising steps for defining a training environment for each RL model using the plurality of training datasets, in accordance with some embodiments of the present disclosure.

FIG. 10 shows an exemplary training strategy of the set of RL models, in accordance with some embodiments of the present disclosure.

FIG. 11 is a flowchart comprising steps for generative design approach to generate multiple material and its manufacturing process designs for a given requirement, in accordance with some embodiments of the present disclosure.

FIG. 12 shows RL model architecture adopted for hot rolled steel sheet design, in accordance with some embodiments of the present disclosure.

FIG. 13 shows design solutions generated by the RL model architecture adopted for hot rolled steel sheet design of FIG. 12, for a given requirement, in accordance with some embodiments of the present disclosure.

FIG. 14 shows comparison results of the design solutions generated by the RL model architecture adopted for hot rolled steel sheet design of FIG. 12, and the domain experts, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Materials are designed for a specific application which requires a set of target properties. While composition selection is a starting point of materials design, it is not independent of its manufacturing route as the changes imparted by the manufacturing route on its structure would determine the final properties of the material. The composition of the material plays a key role during the manufacturing process as the material evolves into the final component which imparts the properties desired. Hence materials are often co-designed along with the manufacturing process. There can be multiple manufacturing process routes feasible to develop a material. Each manufacturing process route often involves multiple sequential manufacturing process steps (or alternatively referred hereafter as processing steps or process steps).

For example, a hot rolled steel sheet can be produced through two different manufacturing routes viz., a conventional casting route or a thin slab casting route depending on the final property requirements. The design process to develop a hot rolled steel sheet through the conventional casting route may involve sequential manufacturing process steps such as composition selection followed by various process steps such as casting, reheating, rough rolling, finish rolling, and ROT & coiling whereas for the thin slab casting route, rough rolling step is not present. Hence, to design the final target material, it becomes imperative to consider the composition of the material and the steps in its manufacturing process together with objectives on its target properties, the cost, the manufacturability etc. in addition to various constraints arriving from environment, social, & governance (ESG) norms, production capabilities, and so on. This generally involves extensive experimentation, characterization and testing to arrive at the desired materials and process design satisfying the required target conditions.

Below are the major challenges and complexities associated with automating the materials and manufacturing process design:

- The properties achieved by the material depend on both the composition and the structure. The structure depends on processing steps and processing parameters. Therefore, both composition and manufacturing processes need to be designed to achieve the required properties.
- The materials and their manufacturing process design is a vast design space problem due to the variable space and combinations.
- Multiple processing routes can be followed to develop a material for a given requirement.
- Manufacturability, ESG norms, and cost aspects need to be integrated into the design process.
- The composition-process structure-property relations that exist in materials design problems are complex and not well understood.
- Limited availability of high quality and comprehensive materials data that are most often proprietary in nature.
- Considerable time for materials design and development owing to large number of experimental trails required and/or computationally expensive simulation models (empirical/analytical/physics-based) in use.

Most of the conventional automated materials design techniques have evolved from experience/experiments based to traditional empirical methods to more theoretical physics-based models and machine learning techniques. The traditional empirical/semi-empirical models-based design involved extensive experimental trials and utilized the designer's experience and composition-process-structure-property relations known. This technique resulted in a linear exploration of the design space through design of experiments. Automated characterization and property measurement tools got introduced to accelerate experimentation.

The recent frameworks such as ICME and materials genome frameworks combined theoretical physics-based models with computational tools and material informatics. Computational design helps to optimize material selection for specific product designs. Material informatics leverage material databases to build AI/ML models to learn relations. Physics based models have made significant advancements in recent times. These models incorporate multi scale modeling of materials from atomistic, nano, micro scales to macro scales. Materials genome engineering combines high throughput computations (HTC), high throughput experimentation, and big data. HTC is used for generating a pool of material designs and virtual screening of material designs to reduce experimental trials. Data mining and AI or ML techniques are used to improve HTC's efficiency further. However, HTC requires high computational cost and resource requirements.

Designers use a combination of experience, computational models, and extensive experimentations to arrive at the materials and process design for a given requirement. The various technology gaps that exist in the industry are as follows:

- The existing automated materials design systems do not perform an integrated design of material compositions and their manufacturing processing steps. Most of the present methods focus on correlating between composition of the materials and their properties, while every processing step involved may also affect the underlying structure and thereby the properties of the developed material.
- The existing automated materials design systems do not consider multiple manufacturing processing routes feasible.
- The existing automated materials design systems do not integrate manufacturability, ESG norms, cost into the decision-making process.
- The data scarcity associated with specific materials design problems limits the effectiveness of current design systems.

The present disclosure solves the technical problems in the art with the methods and systems for automated design of materials and its manufacturing process for desired properties. The present disclosure addresses this gap by using a multi-agent setup for automated design, wherein a distinct Reinforcement learning (RL) agent is used to mirror the composition selection (CS) and various sequential manufacturing process steps (PS) involved in its manufacturing route. The distinct RL agents learn from both past design data and computational models (empirical/analytical/physics-based models) representing the design process. The present disclosure also integrates other important parameters such as manufacturability, ESG norms, cost, process energy etc. and their relative importance into the design decision making process of the RL agents by expressing them as reward components upon which the RL agents are trained.

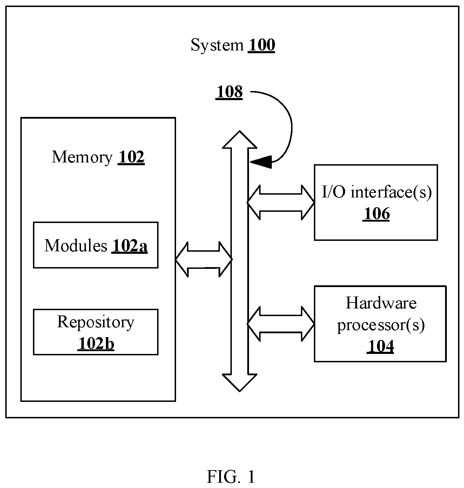

Referring now to the drawings, and more particularly to FIG. 1 through FIG. 14, where similar reference characters denote corresponding features consistently throughout the figures, there are shown preferred embodiments, and these embodiments are described in the context of the following exemplary systems and/or methods.

FIG. 1 is an exemplary block diagram of a system 100 for automated design of materials and its manufacturing process for desired properties, in accordance with some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface (GUI), and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 102 may include any computer-readable medium known in the art including, for example, volatile memory, such as static random-access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. In an embodiment, the plurality of modules 102a can include various sub-modules (not shown in FIG. 1). Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external databases may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

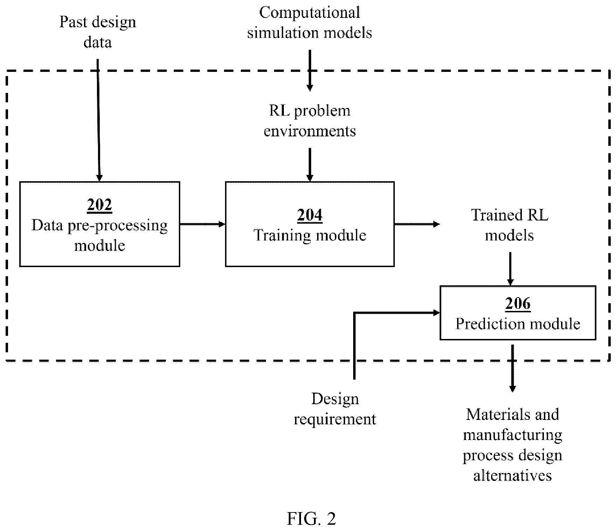

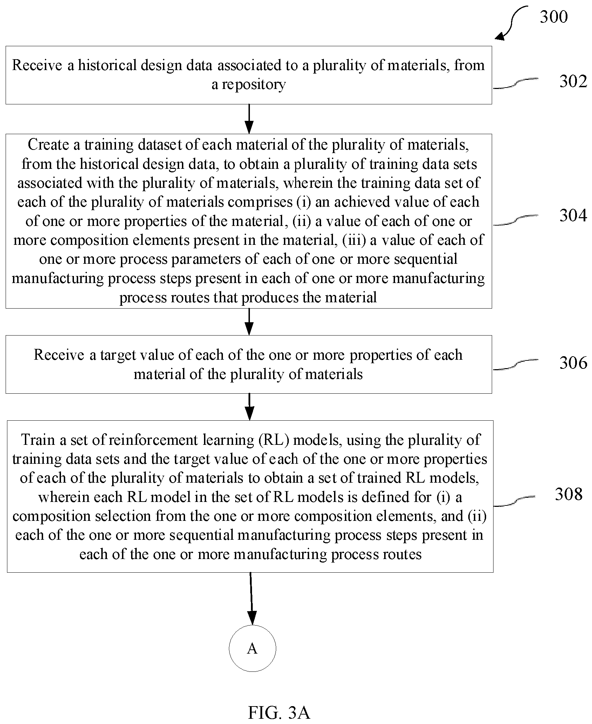

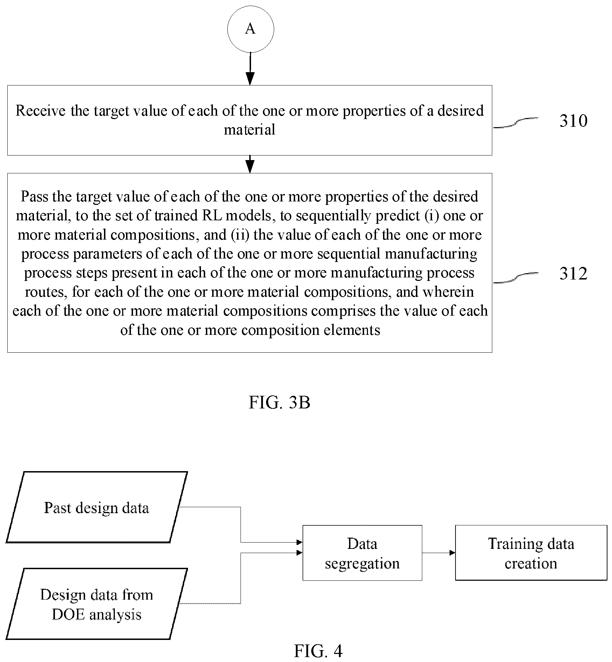

Referring collectively to FIG. 2 and FIGS. 3A-3B, components and functionalities of the system 100 are described in accordance with an example embodiment of the present disclosure. For example, FIG. 2 is an exemplary block diagram illustrating the plurality of modules 102a of the system 100 of FIG. 1, for automated design of materials and its manufacturing process for desired properties, in accordance with some embodiments of the present disclosure. In an embodiment, the plurality of modules 102a include a data pre-processing module 202, a training module 204, and a prediction module 206.

For example, FIGS. 3A-3B illustrate exemplary flow diagrams of a processor-implemented method 300 for automated design of materials and its manufacturing process for desired properties, using the system 100 of FIG. 1, in accordance with some embodiments of the present disclosure. Although steps of the method 300 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 302 of method 300, the one or more hardware processors 104 of the system 100 are configured to receive historical design data associated to a plurality of materials, from a repository 102b. In an embodiment, the historical design data includes past design data and/or the design data that is generated from design of experiments (DOE) analysis. The historical design data is stored in repository 102b.

More specifically the historical design data of each material includes a composition, number and proportions of the composition elements present in the composition, the manufacturing route employed, the number of manufacturing process steps present in the manufacturing route employed, process parameters of each manufacturing process step, achieved properties of the material, other parameters considered during the manufacturing process, and so on.

At step 304 of the method 300, the one or more hardware processors 104 of the system 100 are configured to create a training dataset of each material of the plurality of materials, from the historical design data received at step 302 of the method 300. A plurality of training data sets corresponding to the plurality of materials is obtained as output of this step. In this step, the historical design data received at step 302 of the method 300 is pre-processed to obtain the plurality of training data sets. In an embodiment, the data pre-processing module 202 is configured to pre-process and create the plurality of training data sets corresponding to the plurality of materials from the historical design data.

FIG. 4 is a flowchart comprising steps for creating a plurality of training datasets of a plurality of materials, from the historical design data, in accordance with some embodiments of the present disclosure. As shown in FIG. 4, pre-processing the historical design data includes but is not limited to data segregation, filling missing data, data normalization, and outlier removal. After the data pre-processing, the training data contains the plurality of training data sets corresponding to the plurality of materials.

The training data set of each material includes (i) an achieved value of each of one or more properties of a material, (ii) a value of each of one or more composition elements present in the material, (iii) a value of each of one or more process parameters of each of one or more sequential manufacturing process steps present in each of one or more manufacturing process routes that produces the material.

In an embodiment, the one or more properties of the material indicate material properties including but are not limited to a set of mechanical properties such as a yield strength (YS), an ultimate tensile strength (UTS), and a percentage of uniform elongation, and other specific properties such as a fracture toughness. The achieved value of each property of the material includes the property value of each property that the material exhibits after its production.

In an embodiment, the one or more composition elements present in the material indicate the elements that are part of the composition of the given material. For example, carbon (C), silicon (Si), iron (Fe), chromium (Cr), and nickel (Ni) are some of the elements (referred to as composition elements) in a material composition. The value of each of one or more composition elements refers to the composition value, a proportion value, or a percentage value of each such composition element.

In an embodiment, the one or more manufacturing process routes indicate the manufacturing process routes that are followed/executed for making such material. There may be a number of manufacturing process routes for making a single material. Further, the one or more sequential manufacturing process steps present in each manufacturing process route indicate the manufacturing process steps that are present in a given single route. Naturally, the manufacturing process steps are executed in a sequential manner (one process after the other in a sequential order), hence, they are called the sequential manufacturing process steps.

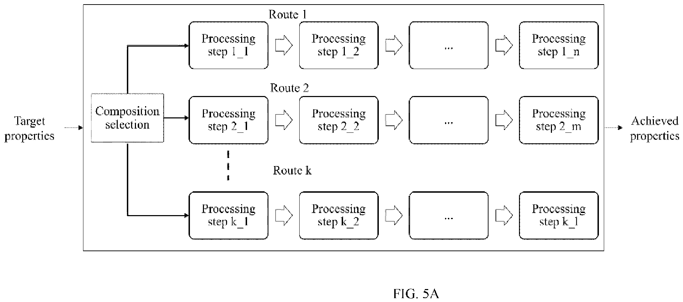

FIG. 5A shows an exemplary design framework for automated design of materials and manufacturing process designs for targeted requirements, in accordance with some embodiments of the present disclosure. The material and its manufacturing process design consists of selecting the composition of the material (composition selection) followed by design of various subsequent processing steps to achieve the specified target properties and desired characteristics. The materials can be developed through multiple manufacturing process routes which indicates a difference in the processing steps involved. The design framework augments the current design process by providing multiple design alternatives to the designer for a given requirement.

As shown in FIG. 5A, the composition selection is a prior step before the manufacturing process route to design any material. There are k number of manufacturing process routes (route 1, route 2,..., route k) defined where each route contain some manufacturing process steps. For example, in route 1 of FIG. 5A, there are n number of manufacturing process steps (process step 1_1, process step 1_2, ..., process step 1_n).

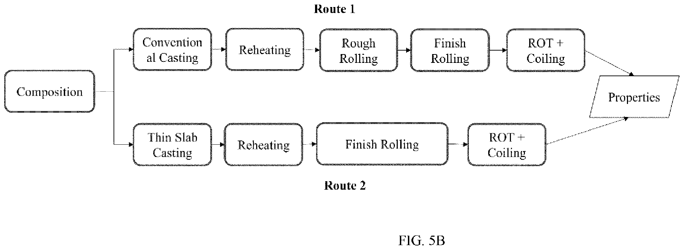

FIG. 5B shows exemplary conventional manufacturing process routes for producing a hot rolled steel sheet. As shown in FIG. 5B, there are two manufacturing process routes (route 1 and route 2) possible to produce the material exhibiting the desired properties. In manufacturing process route 1, the one or more sequential manufacturing process steps are conventional casting, reheating, rough rolling, finish rolling, and ROT & coiling. Because these manufacturing process steps are sequential, the first manufacturing process step (after the composition selection) in route 1 is the conventional casting, next the reheating, and so on the last manufacturing process step is the ROT & coiling. Similarly, the one or more sequential manufacturing process steps in the manufacturing process route 2 are thin slab casting, reheating, finish rolling, and ROT & coiling.

The one or more process parameters of each sequential manufacturing process step indicate the process parameters defined for each sequential manufacturing process step based on the nature of the corresponding manufacturing process. The number of parameters and the variety of parameters may not be same for each sequential manufacturing process step. For example, the process parameters of the reheating process step are a holding temperature, a heating rate, and a holding time. Similarly, the process parameters for the thin slab casting process step are a casting speed, a melt superheat, a nozzle diameter, and a cooling rate. The value of each process parameter corresponding to the sequential manufacturing process step indicates the parameter value in a given measurement units.

At step 306 of the method 300, the one or more hardware processors 104 of the system 100 are configured to receive a target value of each of the one or more properties of each material of the plurality of materials. The target value of each property of the material refers to the target property value of each property that the given material should have. In an embodiment, the target value of each property of the material is provided randomly based on a strategy defined by a material designer or a user.

At step 308 of the method 300, the one or more hardware processors 104 of the system 100 are configured to train a set of reinforcement learning (RL) models, to obtain a set of trained RL models. The plurality of training data sets obtained at step 304 of the method 300 and the target value of each of the one or more properties of each material received at step 306 of the method 300. In an embodiment, the training module 204 is configured to train the set of reinforcement learning (RL) models with the plurality of training data sets to obtain the set of trained RL models.

Each RL model is independent, and the specific RL algorithm and architecture of neural networks used (number of hidden layers, activation function, etc.) can be chosen appropriately for the given manufacturing process step/composition selection problem. Each RL model in the set of RL models is defined for (i) a composition selection that contain information about one or more composition elements present in the corresponding material, and (ii) each of the one or more sequential manufacturing process steps present in each manufacturing process route. For example, to follow the manufacturing process route 1 of FIG. 5B, a set of 6 RL models are defined where one RL model is defined for the composition selection, and one each of the remaining 5 RL models are defined for the conventional casting, the reheating, the rough rolling, the finish rolling, and the ROT & coiling. Similarly, to follow the manufacturing process route 2 of FIG. 5B, a set of 5 RL models are defined where one RL model is defined for the composition selection, and one each of the remaining 4 RL models are defined for the thin slab casting, the reheating, the finish rolling, and the ROT & coiling.

Thus, the framework of the present disclosure employs the RL models (interchangeably referred to as RL agents) for taking the various decisions in the design problem. The model architecture of the present disclosure consists of a modular multi-agent setup for automated design, wherein a distinct RL model is assigned to make decisions on the composition selection (CS) and various sequential manufacturing process steps (PS) involved in its manufacturing process route. Each RL model is a distinct RL agent and therefore the terms 'RL model' and 'RL agent' are used interchangeably in this description. The RL agents work in a sequential fashion to generate the material composition and manufacturing process parameters for each of the manufacturing process steps present in the given manufacturing process route for a given target requirement. The RL agents have an inherent hierarchy as per the position of the processing step in the design process.

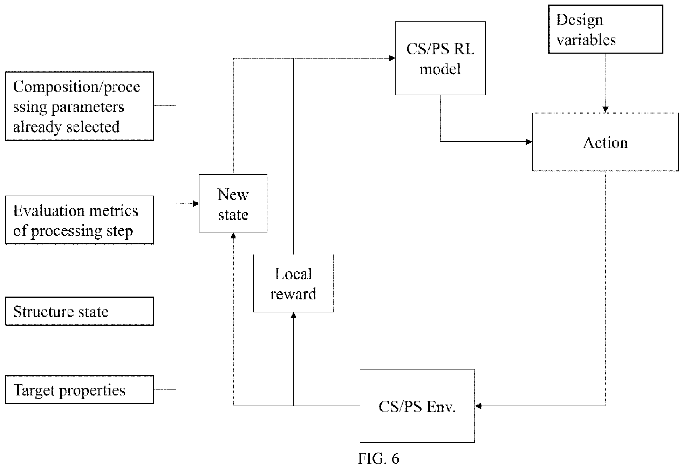

FIG. 6 shows an exemplary setup of the RL problem for a materials design decision making problem, in accordance with some embodiments of the present disclosure. Referring to FIG. 6, the composition selection (CS) / manufacturing processing step (PS) problem can be configured as an RL problem as follows. An RL problem consists of an agent learning a policy by working with an environment. For a given state condition, the agent performs actions on the environment and the environment provides the next state, reward, and done information accordingly. In the case of a design decision problem such as the composition selection (CS) or processing step (PS) in a material and their manufacturing process design, the environment is composed of one or more simulation model(s) for the specified process. The simulation model can be an analytical model, empirical model, machine learning (ML) model, or any other type of physics-based model. For example, a casting process environment can be composed of an empirical casting model which can evaluate the microstructure of the cast after casting process if the composition of the material and the casting process variables are given. The state of a CS/PS environment consist of the following in the present disclosure:

1. The composition/process parameters (if relevant) of the upstream decision problem already selected.
2. Evaluation metrics of the processing step. This can be composed of one or more sustainability indices, cost, process/equipment constraints, and structure state constraints related to the processing step.
3. Structure state variables: these are parameters that specify the current state of the structure of the material.
4. The target properties: the final property values to be achieved which is part of the requirements for the materials and manufacturing process design.

The actions of the RL agent for a CS/PS consist of various design variables of the problem. In the case of a CS, the composition values of the elements in the material constitute the action. In the case of a PS, the design variables consist of the process parameters of the specified process. For example, for the reheating process, the design variables can be holding time and holding temperature. The reward provided by a CS/PS environment is the local reward component of the total reward that the RL agent receives.

Thus, the rewards of these RL agents consist of two components: local rewards (the reward of the specific CS/PS problem) and global rewards. The local rewards take account of the performance of the agent based on the set of criteria specific to the process under consideration. The global reward accounts for the satisfaction of target properties and desired overall characteristics of the material. Upon training based on this reward setup, an RL agent learns to set the process variables of the given decision process to satisfy both the process specific expectations and contribute to the overall properties. If some of the decision problems are common to multiple processing routes, the same RL agent can be used for the problems in all the routes. However, the RL problem needs to be configured accordingly.

In the case of a CS problem, the local reward consists of one or more of:

1. Composition dependent sustainability indices such as elemental extraction energy and a carbon footprint.
2. Cost of the material based on composition.
3. Material properties solely dependent on composition.

In the case of a PS, the local rewards consist of one or more of the following:

1. Process specific contributions towards sustainability indices. For example, energy consumption during a rolling process.
2. Process specific contributions to cost.
3. Intermediate parameter constraints: It includes one or more of

a. Material structure state constraints such as the maximum limit on the grain size after a given processing step.
b. Process/equipment related constraints such as limiting the maximum rolling force below a given limit in rolling.
c. Process end state variable constraints.

The overall properties of the designed material are evaluated by a property evaluator based on the composition and the microstructure. The global rewards are evaluated based on the satisfaction of property targets and the values of desired properties. For example, the property targets might be values of a set of mechanical properties, like yield strength (YS), ultimate tensile strength (UTS), and % uniform elongation, to be achieved by the material. However, the fracture toughness can be a property that is desired. In such a case, a positive reward can be given proportional to the value of achieved fracture toughness.

More specifically the state of each RL model of the composition selection, and each sequential manufacturing process step, is defined with respect (i) the value of each of the one or more process parameter of each of the one or more sequential manufacturing process steps that are precedent to the corresponding sequential manufacturing process step present in each manufacturing process route, (ii) the value of each of the one or more composition elements, (iii) the target value of each of one or more properties of the material, (iv) the one or more evaluation metrics, and (v) one or more material structure state parameters.

The action of each RL model of the composition selection, and each sequential manufacturing process step, is defined with respect to the value of each of one or more composition elements present in the material, the value of each of the one or more process parameters of the corresponding sequential manufacturing process step present in each manufacturing process route, respectively. The reward (or the total reward) of each RL model is defined as a sum of the value of the local reward function of the corresponding RL model and the value of the global reward function.

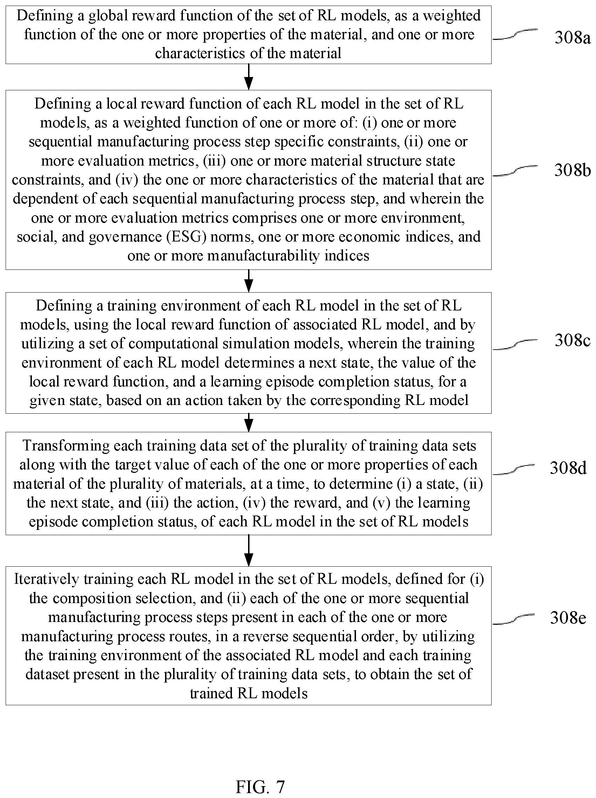

FIG. 7 is a flowchart comprising steps for training the set of RL models, using the plurality of training data sets to obtain the set of trained RL models, in accordance with some embodiments of the present disclosure. As shown in FIG. 7, training the set of RL models, using the plurality of training data sets to obtain the set of trained RL models is further explained through steps 308a to 308e.

At step 308a, a global reward function of the set of RL models is defined. The global reward function is defined as a weighted function of the one or more properties of the material, and one or more characteristics of the material.

At step 308b, a local reward function of each RL model in the set of RL models, is defined. The local reward function of each RL model is defined a weighted function of one or more of: (i) one or more sequential manufacturing process step specific constraints, (ii) one or more evaluation metrics, (iii) one or more material structure state constraints, and (iv) the one or more characteristics of the material that are dependent of each sequential manufacturing process step. The one or more evaluation metrics include one or more environment, social, and governance (ESG) norms, one or more economic indices, and one or more manufacturability indices.

The weighted function of both the local reward function and the global reward function indicates the relative weights of each parameter present in the corresponding weighted function. The relative importance of various reward components is set through weights assignment. The objective here is to capture the relative importance of variables, make each agent rewarded a competing mix of local and global rewards, and thereby enforce the problem constraints and satisfy the target properties effectively.

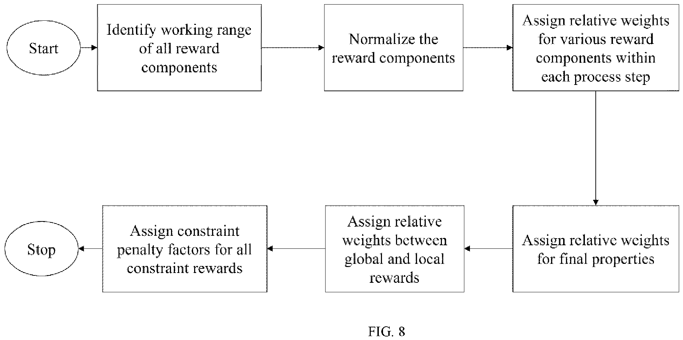

FIG. 8 is a flowchart comprising steps for normalizing and assigning relative weights to reward components, in accordance with some embodiments of the present disclosure. As shown in FIG. 8, the initial steps involve analyzing the working range (lower and upper limits) of all reward components and normalizing each component based on the working range. The working range can be estimated by carrying out the design of experiments (DoE) analysis in the design space of the material and its manufacturing process design problem. Alternatively, it can be determined from past data, experience, and the allowable ranges of various composition and processing variables. The normalizing can be done to a range of 0 to 1 assuming each parameter to be uniformly distributed between the identified working ranges.

There are four types of weights which can be assigned as given below:

1. Relative weights for various reward components within a CS/PS: This weight assignment incorporates the relative importance of various sustainability indices, cost, and intermediate parameter constraints which is set based on experience.
2. Relative weights for final properties (YS, UTS, etc.): If achieving some of the final properties is more important than others, it can be captured using these weights.
3. Relative weights between local and global rewards: This signifies the weightage given to CS/PS specific rewards and rewards corresponding to overall desirable properties achieved. This does not involve the various constraints of specific CS/PS problems.
4. Relative constraint penalty factors to set importance and relevance of various problem constraints.

In the present disclosure, the reward components for specific constraints of the problem are expressed as fuzzy functions. The constraints include process step specific constraints such as 'grain size at the end of casting' should be less than a given value and overall property targets such as 'yield strength' should be greater than a given value, and so on. The constraints can be of two different extremes, either a lower limit or an upper limit. For an upper limit (higher limit) constraint, i.e. the parameter (x) value should be less than the maximum value ($x_{ul}$), a negative reward is assigned when the constraint is not satisfied, i.e. $x >= x_{ul}$. Then the functional representation of the reward component for such specific constraint is given as,

$$f_{ul}(x) = \begin{cases} 0 & if \ x \leq (1-\alpha)x_{ul} \\ -1 & if \ x \geq (1+\alpha)x_{ul} \\ \dfrac{(1-\alpha)x_{ul}-x}{2\alpha x_{ul}} & if \ x > (1-\alpha)x_{ul} \ and \ x < (1+\alpha)x_{ul} \end{cases}$$

For a lower limit constraint, i.e. the parameter (x) value should be more than the minimum value ($x_{ll}$), a negative reward is assigned when the constraint is not satisfied, i.e. $x <= x_{ll}$. Then the functional representation of the reward component for such specific constraint is given as,

$$f_{ll}(x) = \begin{cases} 0 & if\ x \geq (1+\alpha)x_{ll} \\ -1 & if\ x \leq (1-\alpha)x_{ll} \\ \dfrac{x-(1-\alpha)x_{ll}}{2\alpha x_{ll}} - 1 & if\ x > (1-\alpha)x_{ll}\ and\ x < (1+\alpha)x_{ll} \end{cases}$$

Where, $\alpha$ is the limit of the fuzzy zone. For example, for a fuzzy limit of $\pm f_x$% with respect to either the upper or lower limit, the value of alpha ($\alpha$) is given as,

$$\alpha = \frac{f_x}{100}$$

For certain parameters, the reward is based upon the normalized value of the parameter with respect to a maximum achievable value. For such parameters, the requirement is that the value should be as low as possible. The normalization reward function is given as,

$$f_{norm}(x, x_{max}) = -\frac{x}{x_{max}}$$

In an embodiment, the local reward function of each RL model associated to the composition selection consists of normalized elemental extraction energy, carbon footprint, cost, carbon equivalent, and corrosion loss. Hence, the local reward function ($LR_{CS}$) of the RL model associated to the composition selection is mathematically represented as:

$$LR_{CS} = wt_{eee}^{CS} * f_{norm}(EEE_{CS}, EEE_{max}^{CS}) + wt_{cfp}^{CS} * f_{norm}(CFP_{CS}, CFP_{max}^{CS}) + wt_{cost}^{CS}$$
$$* f_{norm}(Cost_{CS}, Cost_{max}^{CS}) + wt_{ce}^{CS} * f_{norm}(CE_{CS}, CE_{max}^{CS}) + wt_{crloss}^{CS}$$
$$* f_{norm}(CRloss_{CS}, CRloss_{max}^{CS})$$

Where, $EEE_{CS}$ is the elemental extraction energy, $CFP_{CS}$ is the carbon footprint, $Cost_{CS}$ is the cost of composition elements, $CE_{CS}$ is the carbon equivalent which determines the weldability, and $CRloss_{CS}$ is the corrosion loss. In an embodiment, exemplary values of the weights are given as follows,

$$wt_{eee}^{CS} = 0.1$$

$$EEE_{max}^{CS} = 2862.58$$

$$wt_{cfp}^{CS} = 0.1$$

$$CFP_{max}^{CS} = 246.2455$$

$$wt_{cost}^{CS} = 0.25$$

$$Cost_{max}^{CS} = 56.0239$$

$$wt_{ce}^{CS} = 0.15$$

$$CE_{max}^{CS} = 0.625$$

$$wt_{crloss}^{CS} = 0.15$$

$$CRloss_{max}^{CS} = 175.4125$$

In an embodiment, the local reward function of each RL model associated to the casting manufacturing process step consists of an upper limit constraint for grain size and secondary dendritic arm spacing obtained after casting. The fuzzy limit considered for the calculation of the local reward is $\pm 10\,\%$. Hence, the local reward function ($LR_{casting}$) of the RL model associated to the casting is mathematically represented as:

$$LR_{casting} = wt_{gs}^{casting} * f_{ul}\left(GS_{casting}\right) + wt_{sdas}^{casting} * f_{ul}(SDAS_{casting})$$

Where, $GS_{casting}$ is the grain size and $SDAS_{casting}$ is the secondary dendritic arm spacing after casting. The exemplary values of the weights are given as follows,

$$wt_{gs}^{casting} = 2.0$$

$$wt_{sdas}^{casting} = 2.0$$

In an embodiment, the local reward function of each RL model associated to the reheating manufacturing process step consists of an upper limit constraint for grain size and a normalized energy required for reheating. The fuzzy limit considered for the calculation of the local reward is $\pm 10\,\%$. Hence, the local reward function ($LR_{Reheating}$) of each RL model associated to the reheating is mathematically represented as:

$$LR_{Reheating} = wt_{gs}^{reheating} * f_{ul}\left(GS_{reheating}\right) + wt_{E}^{reheating}$$
$$* f_{norm}(E_{reheating}, E_{max}^{reheating})$$

Where, $GS_{reheating}$ is the austenite grain size after reheating, and $E_{reheating}$ is the energy consumed during reheating. The exemplary values of the weights are given as follows,

$$wt_{gs}^{reheating} = 4.0$$

$$wt_{E}^{reheating} = 0.5$$

$$E_{max}^{reheating} = 1000$$

In an embodiment, the local reward function of each RL model associated to the rough rolling manufacturing process step consists of an upper limit constraint for grain size and roll force at each roll pass, lower limit constraint for rough rolling exit temperature and a normalized energy required for rough rolling. The fuzzy limit considered for the calculation of the local reward is $\pm 10\,\%$. The local reward function ($LR_{RR}$) of the RL model associated to the rough rolling is mathematically represented as:

$$LR_{RR} = wt_{gs}^{RR} * f_{ul}(GS_{RR}) + wt_{rrt}^{RR} * f_{ll}(RRT_{RR}) + wt_{E}^{RR} * f_{norm}(E_{RR}, E_{max}^{RR}) +$$
$$\sum_{i=1}^{n_{RR}} wt_{rf}^{RR} * f_{ul}(RF_{i}^{RR})$$

Where, $n_{RR}$ is the number of roll passes, $GS_{RR}$ is the austenite grain size, $RRT_{RR}$ is the rough rolling exit temperature, $E_{RR}$ is the energy consumed for deformation and $RF_{i}^{RR}$ is the roll force corresponding to the roll pass $i$. The exemplary values of the weights are given as follows,

$$wt_{gs}^{RR} = 3.2$$

$$wt_{rrt}^{RR} = 2.0$$

$$wt_{E}^{RR} = 0.5$$

$$E_{max}^{RR} = 11.0$$

$$wt_{rf}^{RR} = 7.5$$

In an embodiment, the local reward function of each RL model associated to the finish rolling manufacturing process step consists of an upper limit constraint for grain size, exit strip velocity, and roll force at each roll pass, lower limit constraint for finish rolling exit temperature and a normalized energy required for finish rolling. The fuzzy limit considered for the calculation of the local reward is $\pm 10\,\%$. The local reward function ($LR_{FR}$) of each RL model associated to the finish rolling is mathematically represented as:

$$LR_{FR} = wt_{gs}^{FR} * f_{ul}(GS_{FR}) + wt_{vel}^{FR} * f_{ul}(v_{FR}) + wt_{frt}^{FR} * f_{ll}(FRT_{FR})$$

$$+ wt_{E}^{FR} * f_{norm}(E_{FR}, E_{max}^{FR}) + \sum_{i=1}^{n_{FR}} wt_{rf}^{FR} * f_{ul}(RF_{i}^{FR})$$

Where, $n_{FR}$ is the number of roll passes, $GS_{FR}$ is the austenite grain, $v_{FR}$ is the exit strip velocity, $FRT_{FR}$ is the finish rolling exit temperature, $E_{FR}$ is the energy consumed for deformation and $RF_{i}^{FR}$ is the roll force corresponding to the roll pass i. The exemplary values of the weights are given as follows,

$$wt_{gs}^{FR} = 3.2$$

$$wt_{vel}^{FR} = 2.0$$

$$wt_{frt}^{FR} = 7.5$$

$$wt_{E}^{FR} = 0.5$$

$$E_{max}^{FR} = 11.0$$

$$wt_{rf}^{FR} = 7.5$$

In an embodiment, the local reward function of each RL model associated to the ROT & coiling consists of an upper limit constraint for grain size, and pearlite interlamellar spacing, and a lower limit constraint for ferrite volume fraction and coiling temperature. The fuzzy limit considered for the calculation of the local reward is $\pm 10\,\%$. The local reward function ($LR_{rot}$) of each RL model associated to the ROT & coiling is mathematically represented as:

$$LR_{rot} = wt_{gs}^{ROT} * f_{ul}(GS_{ROT}) + wt_{isl}^{ROT} * f_{ul}(ILS_{ROT}) + wt_{vf}^{ROT} * f_{ll}(VF_{ROT})$$

$$+ wt_{ct}^{ROT} * f_{ll}(CT_{ROT})$$

Where, $GS_{ROT}$ is the grain size of ferrite, $ILS_{ROT}$ is the pearlite interlamellar spacing, $VF_{ROT}$ is the volume fraction of ferrite, and $CT_{ROT}$ is the coiling temperature. The exemplary values of the weights are given as follows,

$$wt_{gs}^{ROT} = 3.2$$

$$wt_{isl}^{ROT} = 3.2$$

$$wt_{vf}^{ROT} = 3.2$$

$$wt_{ct}^{ROT} = 7.5$$

In an embodiment, the global reward function is defined based upon the achieved properties with respect to the design requirements. The global reward function consists of a lower limit constraint for yield strength, ultimate tensile strength, % uniform elongation, and normalized fracture toughness of the final component. The fuzzy limit considered for the calculation of the local reward is $\pm 10$ %. The global reward (GR) is given as,

$$GR = wt_{ys} * f_{ll}(YS) + wt_{uts} * f_{ll}(UTS) + wt_{elon} * f_{ll}(Elon) + wt_{k1c}$$
$$* f_{norm}(K1c, K1c_{max})$$

Where, YS is the yield strength, $UTS$ is the ultimate tensile strength, $Elon$ is the % uniform elongation, and $K1c$ is the fracture toughness. The exemplary values of the weights are given as follows,

$$wt_{ys} = 10.0$$

$$wt_{uts} = 10.0$$

$$wt_{elon} = 10.0$$

$$wt_{k1c} = 1.0$$

$$K1c_{max} = 600$$

In an embodiment, the total reward is defined as the sum of all the local rewards and the global reward. A predefined relative weight is provided to all local reward components excluding the constraints. In an embodiment, an exemplary predefined relative weight provided for all local reward components excluding the constraints is 0.25. Similarly, a predefined relative weight is provided to the global reward components. In an embodiment, the predefined relative weight provided to the global reward components is 1.0.

$$wt_{LR} = 0.25$$

The relative importance of various constraints of the problem are set through a constraint stiffness (constraint penalty factor). The constraint stiffness for the local rewards is given as follows,

$$st_{FRT} = 7.5$$

$$st_{RF} = 7.5$$

$$st_{CT} = 7.5$$

$$st_{others} = 4.0$$

The constraint stiffness for the roll force ($st_{RF}$) for both rough and finish rolling, FRT ($st_{FRT}$) in finish rolling & coiling temperature ($st_{CT}$) constraint is taken as 7.5 and for all other cases it is taken as 4.0. As the global reward considers the properties for which the design is done, for all the design requirements, a constraint stiffness is given the highest weightage (for example, 10.0), giving it the highest weightage, as shown below,

$$st_{ys} = 10.0$$

$$st_{uts} = 10.0$$

$$st_{elon} = 10.0$$

At step 308c, a training environment for each RL model is defined, using the local reward function of corresponding RL model, and by utilizing a set of computational simulation models. The training environment of each RL model determines a next state, the value of the local reward function, and a learning episode completion status, for a given state, based on an action taken by the corresponding RL model.

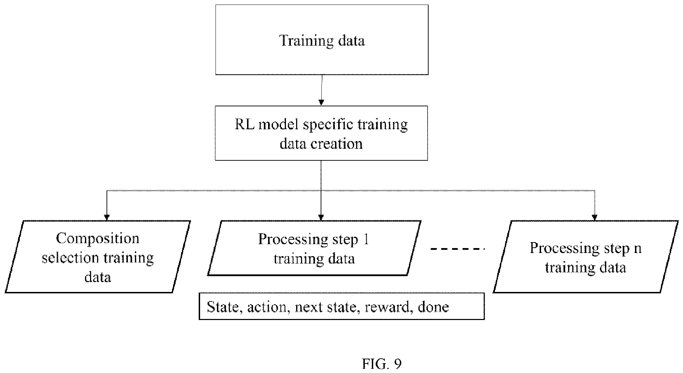

At step 308d, each training data set of the plurality of training data sets, along with the target value of each of the one or more properties of each material of the plurality of materials, is transformed to determine (i) the state, (ii) the next state, and (iii) the action, (iv) the reward, and (v) the learning episode completion status, of each RL model. FIG. 9 is a flowchart comprising steps for defining the training environment for each RL model using the plurality of training datasets, in accordance with some embodiments of the present disclosure. As shown in FIG. 9, each training dataset is transformed into learning experiences upon which the RL agents can learn. The learning experiences comprises the RL model specific training data or in other words, the training data for the composition selection (CS) and the training data for each manufacturing process step (PS). In an embodiment, a data transformation module (202a) (not shown in FIG. 2) of the data pre-processing module 202 is configured to transform each training data set into (i) the state, (ii) the next state, and (iii) the action, (iv) the reward, and (v) the learning episode completion status, for each RL model.

At step 308e, each RL model defined for (i) the composition selection, and (ii) each of the one or more sequential manufacturing process steps present in each of the one or more manufacturing process routes, is iteratively trained, by utilizing the training environment of the corresponding RL model and each training dataset present in the plurality of training data sets, to obtain the set of trained RL models. In the present disclosure, each RL model defined for (i) the composition selection, and (ii) each of the one or more sequential manufacturing process steps present in each of the one or more manufacturing process routes, is iteratively trained in a reverse sequential order.

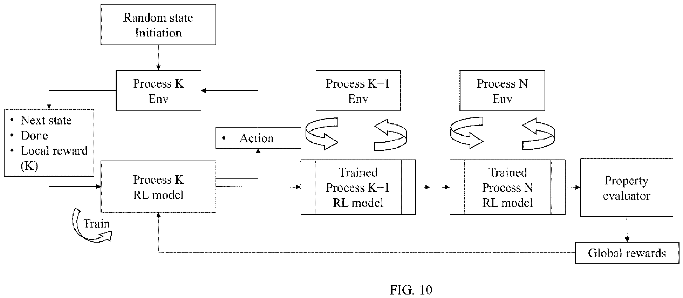

FIG. 10 shows an exemplary training strategy of the set of RL models, in accordance with some embodiments of the present disclosure. As shown in FIG. 10, the RL agents for various CS/PS problems are trained in the reverse sequence. i.e., the last PS RL agent is trained first and sequentially the rest of the agents are trained conditionally utilizing the downstream RL agents as trained prediction models in evaluation mode. For example, in the manufacturing process route 1 of FIG. 5B, the RL agent of the last manufacturing process step 'ROT & coiling' is trained first. Then, the RL agent of previous manufacturing process step 'finish rolling' is trained next, and so on the RL agent of the composition selection is trained at last, to obtain the set of trained RL models of the corresponding composition selection and the manufacturing process steps in each manufacturing process route.

The set of trained RL models obtained at this step can be used in real-time to produce different design solutions in terms of the composition selection and the corresponding process parameters for the desired material with the desired properties. Hence the set of trained RL models make the present disclosure as a generative design approach which can generate multiple material and its manufacturing process design solutions for a given design requirement.

At step 310 of the method 300, the one or more hardware processors 104 of the system 100 are configured to receive the target value of each of the one or more properties of a desired material. The desired material is the material that is required as an outcome of the manufacturing process and the target value of each property of the desired material refers to the target property value of each property that the desired material should have.

At step 312 of the method 300, the one or more hardware processors 104 of the system 100 are configured to pass the target value of each of the one or more properties of the desired material received at step 310 of the method 300, to the set of trained RL models obtained at step 308 of the method 300. The set of trained RL models sequentially predict different design solutions (i) the one or more material compositions, and (ii) the value of each of the one or more process parameter of each of the one or more sequential manufacturing process steps present in each of the one or more manufacturing process routes, for each of the one or more material compositions. Here, each of the one or more material compositions comprises the value of each of the one or more composition elements that contain in the desired material after the manufacturing process. In an embodiment, the prediction module 206 is configured to predict different design solutions by the set of trained RL models.

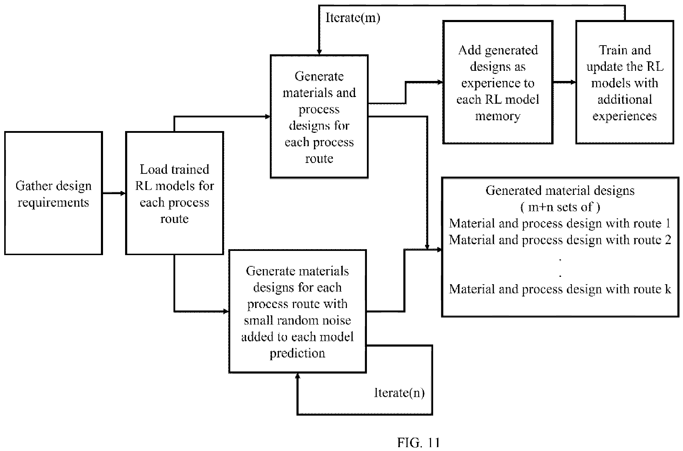

FIG. 11 is a flowchart comprising steps for generative design approach to generate multiple material and its manufacturing process designs for a given requirement, in accordance with some embodiments of the present disclosure. The generative design approach of the present disclosure starts with gathering design requirements from the designer which predominantly involves target values for various identified properties of the materials. The set of trained RL agents are loaded in the next step. Some of the RL agents may be common to multiple processing routes as the decision problem may be common. The two approaches used to generate multiple design solutions for the given requirement are explained below. The first approach is achieved by allowing the agents to learn on the given problem for multiple learning episodes. The second approach is achieved by adding a small noise to the individual agent predictions.

In the first approach, the RL agents are used to predict the composition and processing parameters for identified processing routes separately. Following each instance of agent predictions, the experiences corresponding to individual agents are determined and added to the memory of respective agents in the prescribed format (state, next state, action, reward, done). The agents are trained with additional experience and the updated agents are used for predictions for the same requirement in the next iteration. This process is iterated for the prescribed number of steps to generate multiple sets of material and its manufacturing process designs for the identified process routes. This approach exploits the understanding of reward landscapes of each individual RL agent and the effect of sequential model predictions to generate multiple material and its manufacturing process designs for the same requirement.

In the second approach, a small random gaussian noise is added to each agent's predictions. The material and its manufacturing process designs are generated through sequential prediction of the respective agents for each processing route. This process is iterated for the prescribed number of steps to generate multiple sets of material and its manufacturing process designs for the identified process routes. The approach generates multiple design solutions by slight augmentation of the preferred design space of the learnt agents along with the effect of sequential model predictions. This approach can be applied to RL algorithms with deterministic policies. However, if the policy is stochastic, this approach may not be needed.

As shown in FIG. 11, the generative design approach of the present disclosure predicts (m+n) sets of design solutions each containing material and the process design (process parameters) for k manufacturing process routes. The designer may further validate each set of the design solution by comparing with the target properties of the required material and the achieved properties of the material resulted from each set of the design solution, to choose the design solution sets for the experimentation.

The present disclosure presents a novel generalized framework as the methods and systems for integrated design of materials and their manufacturing process to achieve given target requirements. The proposed design framework employs distinct RL agents to carry out various design process steps such as composition selection and various manufacturing process steps in a specific way. The RL agents in the framework function in a synergistic manner. Each model makes conditional predictions, informed by the output of its preceding step, thus ensuring a coherent and sequential flow of information and decision-making, akin to the original decision process.

The modular approach reduces the complexity of individual models as opposed to a single ML model learning the relationship between various composition and manufacturing process variables, and the final properties, and makes the framework scalable. It also enables integrated design of materials and their manufacturing process considering multiple feasible discrete process routes. The environment for training an RL agent consists of one or more analytical, empirical, or physics-based models to represent the design steps and various calculations of parameters of interest. The usage of RL agents allows the models to learn from both past design data and computational models making the design framework less reliant on data and more efficient.

The various RL agents in the design framework are trained in a sequence opposite to that of the evolution of the material. That is, the RL agent representing the last manufacturing process step is trained first and the rest of the RL agents are trained subsequently step-by-step. While training a given RL agent, the trained RL agents that correspond to the successive steps in the design process, are utilized as learnt predictor models. This type of training makes the agent being trained to be aware of the performance of the succeeding process step agents. This training approach, along with conditional prediction of the models during design stage allows the models to work in tandem in an effective manner to achieve the overall requirements. That is, in the case of a hot rolled steel design, while training the rough rolling RL agent, the succeeding learnt RL agents for finish rolling and ROT & coiling steps are used to predict the designs for the respective process steps. This way, the rough rolling RL agent is aware of the performance of learnt succeeding models. This training method allows the framework to exploit the good performing regions of the RL agents to generate feasible designs for a requirement with limited training.

The RL agents have an inherent hierarchy, because the earlier decisions have a large influence on what is possible for the subsequent agents to accomplish. Therefore a "proprietary" or "novel" reward function is designed. The reward provided for a given process step RL agent is composed of two components: a local reward component and global reward component. The local rewards are given based on the agent's performance in satisfying its process specific goals and constraints while the global reward is given for satisfying the overall requirements (which is dependent on the performance of all the RL agents in the framework). This type of a reward system enables the individual RL models to consider both the process specific constraints and goals along with final property requirements during decision making emulating the scenario of multiple design experts working together in tandem to achieve the overall goal. For example, in the case of hot rolled steel design, for casting process RL models, the rewards are composed of local rewards dependent on casting process constraints such as constraints on the microstructural state at the end of casting, and a global component which is dependent on the final properties such as YS and UTS achieved by the material designed. In addition, the local and global rewards integrate various desirable parameters such as manufacturability, ESG norms, cost, process energy etc. and their relative importance through a weighted approach leading to the design of manufacturable, cost effective and sustainable materials.

A generative design approach of the present disclosure combines two methods to generate multiple material and manufacturing process design alternatives for a given requirement. Multiple design alternatives can be generated for the given requirements for a given processing route and reward weights by (a) allowing the agents to learn on the given requirement condition for multiple learning episodes, and (b) adding a small noise to the individual agent predictions. These methods exploit the reward landscape understanding of the agents and perturbation of the preferred design space respectively. These methods reduce the reliance on individual agent performance levels. It can potentially generate diverse solution alternatives compared to human experts leading to improved design quality and reduced design cycle time.

Further, the integrated design framework of the present disclosure augments the current design process by providing multiple design alternatives to the designer that leads to efficient discovery of composition and manufacturing processes and reduced manual efforts and experimental trials.

A modular multi-agent setup for automated design, wherein a distinct Reinforcement learning (RL) agent is used to mirror the composition selection (CS) and various process steps (PS) involved in its manufacturing. The distinct RL agents inherit the hierarchy involved in the materials and their manufacturing process design. For example, in the case of a hot rolled steel design, distinct RL agents are assigned to make decisions on composition selection, casting, reheating, rolling and ROT. This modular architecture provides the following advantages.

    a. increased flexibility and allows to consider multiple processing routes feasible. The same RL agent can be used to make decisions for a processing step common to multiple processing routes.

    b. allows to train distinct RL agents with process specific data available.

The methods and systems of the present disclosure represents the composition selection and various process step design problems as RL problems and train the RL agents effectively are proposed allowing the models to learn from both past design data and computational models (empirical/analytical/physics based) representing the design process.

Further, the methods and systems of the present disclosure integrate manufacturability, ESG norms, cost, process energy etc. and their relative importance into the design decision making process of the RL agents by expressing them as reward components upon which the RL agents are trained. The method leads to the following benefits:

    a. Reduce the rejection of material designs due to poor manufacturability and failing to satisfy ESG norms.

    b. Cost effective and greener designs.

A generative design methodology to generate multiple alternatives of materials and their manufacturing process designs for a given requirement is presented which provides multiple design options to the designer with potentially larger diversity leading to faster convergence and reduced design cycle time.

The methods and systems of the present disclosure accelerates the materials design life cycle and reduce the experimental trials required through automated generation of multiple candidate material and manufacturing process designs from various feasible processing routes for a given requirement. The methods and systems of the present disclosure can potentially provide diverse and unexplored solutions of materials and manufacturing process designs as compared to expert driven decisions, which are often limited by experiential biases or lack of sufficient exploration.

The various manufacturability, ESG norms, cost, process energy considerations are incorporated into the decision making, leading to the design of manufacturable, cost effective and sustainable materials and manufacturing processes. The methods and systems of the present disclosure uses RL agents to learn from both past design data and computational models making the design framework less reliant on data, practical, and significantly more efficient than the conventional techniques in the art.

Example scenario:

The methods and systems of the present disclosure were tested on the design of hot rolled steel sheets. The design process was considered as in FIG. 5B which contained two different casting routes: conventional casting route (Route 1) and thin slab casting route (Route 2). The major difference between the process routes is the presence of a rough rolling process step in conventional casting route. In addition, the feasible variable ranges of various processing steps vary with the processing routes.

In this design process, the design input consists of target requirements of yield strength (YS), ultimate tensile strength (UTS), % uniform elongation, and sheet thickness. The objective of the design process was to satisfy the target properties along with minimizing/maximizing the following parameters.

    a. Minimize

        i. Energy functions, Carbon footprint, Cost

    b. Maximize
    ii. Weldability, corrosion resistance, fracture toughness

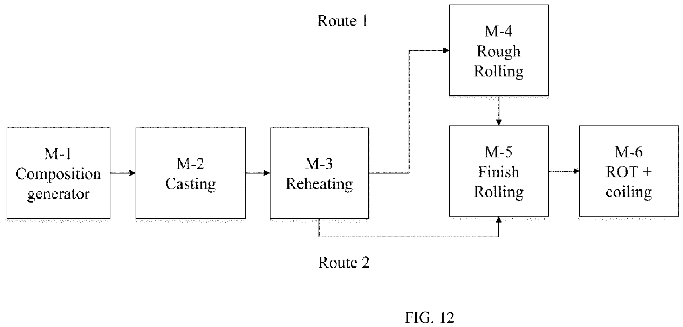

In addition, there are several process specific constraints which need to be satisfied. These constraints relate to process equipment constraints, process end state constraints, and microstructural state constraints which were derived from domain knowledge. For example, in the casting process, the grain size and secondary dendritic arm spacing (SDAS) at the end of casting should be less than the respective limits provided. FIG. 12 shows RL model architecture adopted for hot rolled steel sheet design, in accordance with some embodiments of the present disclosure. The architecture consists of six RL agents which were assigned to composition selection, casting, reheating, rough rolling, finish rolling, and run out table (ROT) & Coiling processes respectively. The rough rolling RL agent was used for conventional casting route only. The casting route was provided as an additional input to train and predict solutions corresponding to a given processing route. The state, actions, and rewards are set up for each of the six decision problems as per the approach presented in the invention. For example, in the case of the Reheating RL agent, the state, actions, and rewards are as given below in line with the state parameters of the reheating process as stated in Table 1, wherein LL means lower limit.

Table 1

| Parameter type | Parameter |
|---|---|
| Target requirements | LL of YS |
| | LL of UTS |
| | LL of % Elongation |
| Composition (in %) | C, Mn, Si, Cr, Ni |
| Casting process design outcomes | Processing route, Casting Grain Size |

- Actions

    ◦ Reheating time
    ◦ Reheating temperature

- Reward components

    ◦ Local reward (process specific)

        - Penalty for violating grain size at the end of reheating.
        - Negative reward proportional to reheating process energy.

    ◦ Global reward (based on final properties achieved)

        - Penalty for not achieving any of the final property targets (function of number of violations and degree of violations)
        - Positive reward proportional to fracture toughness achieved.

The variables involved in the design process are predominantly continuous. Considering these aspects, an off-policy RL algorithm: Deep Deterministic Policy Gradient (DDPG) is the RL algorithm selected for this framework. The DDPG was considered as an exemplary implementation, but any RL algorithm may be substituted as appropriate. Empirical models which represent the various processing steps and property evaluations, are used to create the RL model environments for this design problem. The relative weights of different types adopted for the problem based on domain knowledge and experience are provided for the reheating process and the global reward components.

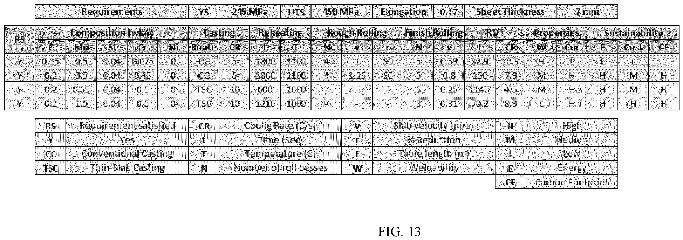

The RL agents are trained as per the training approach presented wherein the ROT & coiling RL agent was trained first and the rest of the agents are trained following the hierarchical sequence keeping the downstream agents as learnt predictor models. A training data was created using the approach presented in the invention, wherein the DoE were performed on the design space of the Materials and process design problem to create a first set of data with inputs consisting of various CS/PS variables and outputs consisting of final achieved properties. The first set of training data was transformed into learning experiences by running it through different input requirement sets and calculating the associated rewards. This was combined with online RL training with random start states to train each RL agent. The generative design approach presented was used to generate Materials and process designs using the learnt models for a given requirement. FIG. 13 shows design solutions generated by the RL model architecture adopted for hot rolled steel sheet design of FIG. 12, for a given requirement, in accordance with some embodiments of the present disclosure.

To evaluate the performance of the developed framework, a benchmark study was carried out with design experts (materials engineers familiar with the hot rolled steel design). In this benchmark study, the design experts and the RL agents were given three sets of different design requirements to work upon. A maximum of 10 design iterations can be performed on a given design problem to come up with the best set of CS/PS variable values to achieve the requirements. The same empirical models were used to evaluate the final properties and process outcomes of the various processing steps. The RL agent was asked to generate 5 materials and process designs each corresponding to the two processing routes to add up to 10 materials and process designs for a given problem following the learning and iterating approach in the generative design strategy. A design score is provided for each design based on the various design goals and constraints defined.

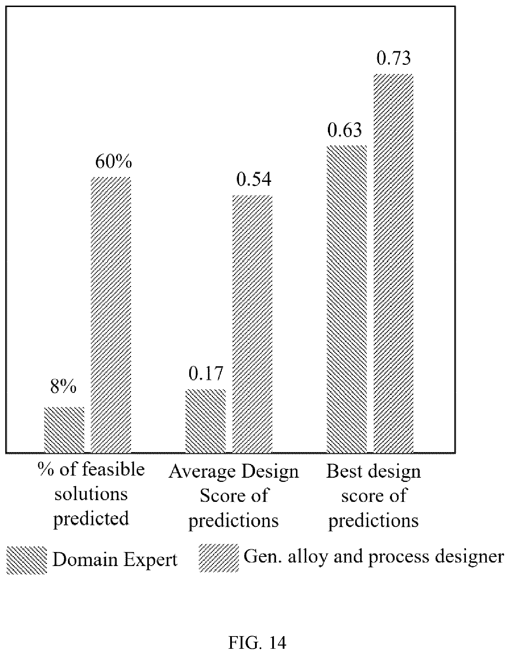

FIG. 14 shows comparison results of the design solutions generated by the RL model architecture adopted for hot rolled steel sheet design of FIG. 12, and the domain expert, in accordance with some embodiments of the present disclosure. As shown in FIG. 14, the comparison shows that the design framework showcased superior performance in terms of design solution performance and design cycle time.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of the present disclosure herein address unresolved problems of automated design of materials and the manufacturing process for the desired properties. The present disclosure integrates other important parameters such as manufacturability, ESG norms, cost, process energy etc. and their relative importance into the design decision making process of the RL agents by expressing them as reward components upon which the RL agents are trained.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor-implemented method (300), comprising:

   receiving, via one or more hardware processors, a historical design data associated to a plurality of materials, from a repository (302);
   creating, via the one or more hardware processors, a training dataset of each material of the plurality of materials, from the historical design data, to obtain a plurality of training data sets associated with the plurality of materials, wherein the training data set of each of the plurality of materials comprises (i) an achieved value of each of one or more properties of the material, (ii) a value of each of one or more composition elements present in the material, (iii) a value of each of one or more process parameters of each of one or more sequential manufacturing process steps present in each of one or more manufacturing process routes that produces the material (304);
   receiving, via the one or more hardware processors, a target value of each of the one or more properties of each material of the plurality of materials (306); and
   training, via the one or more hardware processors, a set of reinforcement learning (RL) models, using the plurality of training data sets and the target value of each of the one or more properties of each of the plurality of materials to obtain a set of trained RL models, wherein each RL model in the set of RL models is defined for (i) a composition selection from the one or more composition elements, and (ii) each of the one or more sequential manufacturing process steps present in each of the one or more manufacturing process routes (308).

2. A processor-implemented method (300) as claimed in claim 1, further comprising:

   receiving, via the one or more hardware processors, the target value of each of the one or more properties of a desired material (310); and
   passing, via the one or more hardware processors, the target value of each of the one or more properties of the desired material, to the set of trained RL models, to sequentially predict (i) one or more material compositions, and (ii) the value of each of the one or more process parameters of each of the one or more sequential manufacturing process steps present in each of the one or more manufacturing process routes, for each of the one or more material compositions, and wherein each of the one or more material compositions comprises the value of each of the one or more composition elements (312).

3. The processor-implemented method (300) as claimed in claim 1, wherein training the set of RL models, using the plurality of training data sets to obtain the set of trained RL models, comprises:

   defining a global reward function of the set of RL models, as a weighted function of the one or more properties of the material, and one or more characteristics of the material (308a);
   defining a local reward function of each RL model in the set of RL models, as a weighted function of one or more of:
   (i) one or more sequential manufacturing process step specific constraints, (ii) one or more evaluation metrics, (iii) one or more material structure state constraints, and (iv) the one or more characteristics of the material that are dependent of each sequential manufacturing process step, and wherein the one or more evaluation metrics comprises one or more environment, social, and governance (ESG) norms, one or more economic indices, and one or more manufacturability indices (308b);

defining a training environment of each RL model in the set of RL models, using the local reward function of associated RL model, and by utilizing a set of computational simulation models, wherein the training environment of each RL model determines a next state, the value of the local reward function, and a learning episode completion status, for a given state, based on an action taken by the associated RL model (308c);

transforming each training data set of the plurality of training data sets along with the target value of each of the one or more properties of each material of the plurality of materials, at a time, to determine (i) a state, (ii) the next state, and (iii) the action, (iv) the reward, and (v) the learning episode completion status, of each RL model in the set of RL models (308d); and

iteratively training each RL model in the set of RL models, defined for (i) the composition selection, and (ii) each of the one or more sequential manufacturing process steps present in each of the one or more manufacturing process routes, in a reverse sequential order, by utilizing the training environment of the associated RL model and each training dataset present in the plurality of training data sets, to obtain the set of trained RL models (308e).

4. The processor-implemented method (300) as claimed in claim 3, wherein:

the state of each RL model of the composition selection, and each sequential manufacturing process step, is defined with respect to one or more of : (i) the value of each of the one or more process parameters of each of the one or more sequential manufacturing process steps that are precedent to the corresponding sequential manufacturing process step present in each manufacturing process route, (ii) the value of each of the one or more composition elements, (iii) the target value of each of one or more properties of the material, (iv) the one or more evaluation metrics, and (v) one or more material structure state parameters,

the action of each RL model of the composition selection, and each sequential manufacturing process step, is defined with respect to the value of each of one or more composition elements present in the material, the value of each of the one or more process parameters of the corresponding sequential manufacturing process step present in each manufacturing process route, respectively, and

the reward of each RL model is defined as a sum of a value of the local reward function of the associated RL model and the value of the global reward function.

5. A system (100), comprising:

a memory (102) storing instructions;
one or more input/output (I/O) interfaces (106); and
one or more hardware processors (104) coupled to the memory (102) via the one or more I/O interfaces (106), wherein the one or more hardware processors (104) are configured by the instructions to:

receive a historical design data associated to a plurality of materials, from a repository;
create a training dataset of each material of the plurality of materials, from the historical design data, to obtain a plurality of training data sets associated with the plurality of materials, wherein the training data set of each of the plurality of materials comprises (i) an achieved value of each of one or more properties of the material, (ii) a value of each of one or more composition elements present in the material, (iii) a value of each of one or more process parameters of each of one or more sequential manufacturing process steps present in each of one or more manufacturing process routes that produces the material;
receive a target value of each of the one or more properties of each material of the plurality of materials; and
train a set of reinforcement learning (RL) models, using the plurality of training data sets and the target value of each of the one or more properties of each of the plurality of materials to obtain a set of trained RL models, wherein each RL model in the set of RL models is defined for (i) a composition selection from the one or more composition elements, and (ii) each of the one or more sequential manufacturing process steps present in each of the one or more manufacturing process routes.

6. The system (100) as claimed in claim 5, wherein the one or more hardware processors (104) are further configured to:

receive the target value of each of the one or more properties of a desired material; and
pass the target value of each of the one or more properties of the desired material, to the set of trained RL models, to sequentially predict (i) one or more material compositions, and (ii) the value of each of the one or more process parameters of each of the one or more sequential manufacturing process steps present in each of the one or more manufacturing process routes, for each of the one or more material compositions, and wherein each of the one or more material compositions comprises the value of each of the one or more composition elements.

7. The system (100) as claimed in claim 5, wherein the one or more hardware processors (104) are configured to train the set of RL models, using the plurality of training data sets to obtain the set of trained RL models, by:

defining a global reward function of the set of RL models, as a weighted function of the one or more properties of the material, and one or more characteristics of the material;

defining a local reward function of each RL model in the set of RL models, as a weighted function of one or more of: (i) one or more sequential manufacturing process step specific constraints, (ii) one or more evaluation metrics, (iii) one or more material structure state constraints, and (iv) the one or more characteristics of the material that are dependent of each sequential manufacturing process step, and wherein the one or more evaluation metrics comprises one or more environment, social, and governance (ESG) norms, one or more economic indices, and one or more manufacturability indices;

defining a training environment of each RL model in the set of RL models, using the local reward function of associated RL model, and by utilizing a set of computational simulation models, wherein the training environment of each RL model determines a next state, the value of the local reward function, and a learning episode completion status, for a given state, based on an action taken by the associated RL model;

transforming each training data set of the plurality of training data sets along with the target value of each of the one or more properties of each material of the plurality of materials, at a time, to determine (i) a state, (ii) the next state, and (iii) the action, (iv) the reward, and (v) the learning episode completion status, of each RL model in the set of RL models; and

iteratively training each RL model in the set of RL models, defined for (i) the composition selection, and (ii) each of the one or more sequential manufacturing process steps present in each of the one or more manufacturing process routes, in a reverse sequential order, by utilizing the training environment of the associated RL model and each training dataset present in the plurality of training data sets, to obtain the set of trained RL models.

8. The system (100) as claimed in claim 7, wherein:

the state of each RL model of the composition selection, and each sequential manufacturing process step, is defined with respect to one or more of : (i) the value of each of the one or more process parameters of each of the one or more sequential manufacturing process steps that are precedent to the corresponding sequential manufacturing process step present in each manufacturing process route, (ii) the value of each of the one or more composition elements, (iii) the target value of each of one or more properties of the material, (iv) the one or more evaluation metrics, and (v) one or more material structure state parameters,

the action of each RL model of the composition selection, and each sequential manufacturing process step, is defined with respect to the value of each of one or more composition elements present in the material, the value of each of the one or more process parameters of the corresponding sequential manufacturing process step present in each manufacturing process route, respectively, and

the reward of each RL model is defined as a sum of a value of the local reward function of the associated RL model and the value of the global reward function.

9. One or more non-transitory machine-readable information storage mediums comprising one or more instructions which when executed by one or more hardware processors cause:

receiving a historical design data associated to a plurality of materials, from a repository;

creating a training dataset of each material of the plurality of materials, from the historical design data, to obtain a plurality of training data sets associated with the plurality of materials, wherein the training data set of each of the plurality of materials comprises (i) an achieved value of each of one or more properties of the material, (ii) a value of each of one or more composition elements present in the material, (iii) a value of each of one or more process parameters of each of one or more sequential manufacturing process steps present in each of one or more manufacturing process routes that produces the material;

receiving a target value of each of the one or more properties of each material of the plurality of materials; and

training a set of reinforcement learning (RL) models, using the plurality of training data sets and the target value of each of the one or more properties of each of the plurality of materials to obtain a set of trained RL models, wherein each RL model in the set of RL models is defined for (i) a composition selection from the one or more composition elements, and (ii) each of the one or more sequential manufacturing process steps present in each of the one or more manufacturing process routes.

10. The one or more non-transitory machine-readable information storage mediums of claim 9, wherein the one or more instructions which when executed by the one or more hardware processors further cause:

receiving the target value of each of the one or more properties of a desired material; and
passing the target value of each of the one or more properties of the desired material, to the set of trained RL models, to sequentially predict (i) one or more material compositions, and (ii) the value of each of the one or more process parameters of each of the one or more sequential manufacturing process steps present in each of the one or more manufacturing process routes, for each of the one or more material compositions, and wherein each of the one or more material compositions comprises the value of each of the one or more composition elements.

11. The one or more non-transitory machine-readable information storage mediums of claim 9, wherein training the set of RL models, using the plurality of training data sets to obtain the set of trained RL models, comprises:

defining a global reward function of the set of RL models, as a weighted function of the one or more properties of the material, and one or more characteristics of the material;
defining a local reward function of each RL model in the set of RL models, as a weighted function of one or more of: (i) one or more sequential manufacturing process step specific constraints, (ii) one or more evaluation metrics, (iii) one or more material structure state constraints, and (iv) the one or more characteristics of the material that are dependent of each sequential manufacturing process step, and wherein the one or more evaluation metrics comprises one or more environment, social, and governance (ESG) norms, one or more economic indices, and one or more manufacturability indices;
defining a training environment of each RL model in the set of RL models, using the local reward function of associated RL model, and by utilizing a set of computational simulation models, wherein the training environment of each RL model determines a next state, the value of the local reward function, and a learning episode completion status, for a given state, based on an action taken by the associated RL model;
transforming each training data set of the plurality of training data sets along with the target value of each of the one or more properties of each material of the plurality of materials, at a time, to determine (i) a state, (ii) the next state, and (iii) the action, (iv) the reward, and (v) the learning episode completion status, of each RL model in the set of RL models; and
iteratively training each RL model in the set of RL models, defined for (i) the composition selection, and (ii) each of the one or more sequential manufacturing process steps present in each of the one or more manufacturing process routes, in a reverse sequential order, by utilizing the training environment of the associated RL model and each training dataset present in the plurality of training data sets, to obtain the set of trained RL models.

12. The one or more non-transitory machine-readable information storage mediums of claim 11, wherein:

the state of each RL model of the composition selection, and each sequential manufacturing process step, is defined with respect to one or more of : (i) the value of each of the one or more process parameters of each of the one or more sequential manufacturing process steps that are precedent to the corresponding sequential manufacturing process step present in each manufacturing process route, (ii) the value of each of the one or more composition elements, (iii) the target value of each of one or more properties of the material, (iv) the one or more evaluation metrics, and (v) one or more material structure state parameters,
the action of each RL model of the composition selection, and each sequential manufacturing process step, is defined with respect to the value of each of one or more composition elements present in the material, the value of each of the one or more process parameters of the corresponding sequential manufacturing process step present in each manufacturing process route, respectively, and
the reward of each RL model is defined as a sum of a value of the local reward function of the associated RL model and the value of the global reward function.

System **100**

**108**

Memory **102**

Modules **102a**

Repository
**102b**

I/O interface(s)
**106**

Hardware
processor(s)
**104**

FIG. 1

Past design
data

Computational
simulation models

RL problem
environments

```
┌─────────────────────────────────────────────────────────────────┐
│                                                                   │
│                                                                   │
│   ┌──────────────────┐      ┌──────────────────┐                  │
│   │       202        │      │       204        │    Trained RL    │
│   │ Data pre-processing ──► │  Training module │ ──►  models       │
│   │     module       │      │                  │                  │
│   └──────────────────┘      └──────────────────┘                  │
│                                              ┌──────────────────┐ │
│                                         ──►  │       206        │ │
│                                              │ Prediction module│ │
│                                              └──────────────────┘ │
└─────────────────────────────────────────────────────────────────┘
```

Design
requirement

Materials and
manufacturing
process design
alternatives

FIG. 2

300

| Receive a historical design data associated to a plurality of materials, from a repository | 302 |

↓

| Create a training dataset of each material of the plurality of materials, from the historical design data, to obtain a plurality of training data sets associated with the plurality of materials, wherein the training data set of each of the plurality of materials comprises (i) an achieved value of each of one or more properties of the material, (ii) a value of each of one or more composition elements present in the material, (iii) a value of each of one or more process parameters of each of one or more sequential manufacturing process steps present in each of one or more manufacturing process routes that produces the material | 304 |

↓

| Receive a target value of each of the one or more properties of each material of the plurality of materials | 306 |

↓

| Train a set of reinforcement learning (RL) models, using the plurality of training data sets and the target value of each of the one or more properties of each of the plurality of materials to obtain a set of trained RL models, wherein each RL model in the set of RL models is defined for (i) a composition selection from the one or more composition elements, and (ii) each of the one or more sequential manufacturing process steps present in each of the one or more manufacturing process routes | 308 |

↓

( A )

FIG. 3A

A

Receive the target value of each of the one or more properties of a desired material ⟶ 310

Pass the target value of each of the one or more properties of the desired material, to the set of trained RL models, to sequentially predict (i) one or more material compositions, and (ii) the value of each of the one or more process parameters of each of the one or more sequential manufacturing process steps present in each of the one or more manufacturing process routes, for each of the one or more material compositions, and wherein each of the one or more material compositions comprises the value of each of the one or more composition elements ⟶ 312

FIG. 3B

Past design data

Design data from DOE analysis

Data segregation

Training data creation

FIG. 4

FIG. 5A

**Route 1**

FIG. 5B

Composition/proce
ssing parameters
already selected

Evaluation metrics
of processing step

Structure state

Target properties

New
state

Local
reward

CS/PS RL
model

Design
variables

Action

CS/PS Env.

FIG. 6

Defining a global reward function of the set of RL models, as a weighted function of the one or more properties of the material, and one or more characteristics of the material

308a

Defining a local reward function of each RL model in the set of RL models, as a weighted function of one or more of: (i) one or more sequential manufacturing process step specific constraints, (ii) one or more evaluation metrics, (iii) one or more material structure state constraints, and (iv) the one or more characteristics of the material that are dependent of each sequential manufacturing process step, and wherein the one or more evaluation metrics comprises one or more environment, social, and governance (ESG) norms, one or more economic indices, and one or more manufacturability indices

308b

Defining a training environment of each RL model in the set of RL models, using the local reward function of associated RL model, and by utilizing a set of computational simulation models, wherein the training environment of each RL model determines a next state, the value of the local reward function, and a learning episode completion status, for a given state, based on an action taken by the corresponding RL model

308c

Transforming each training data set of the plurality of training data sets along with the target value of each of the one or more properties of each material of the plurality of materials, at a time, to determine (i) a state, (ii) the next state, and (iii) the action, (iv) the reward, and (v) the learning episode completion status, of each RL model in the set of RL models

308d

Iteratively training each RL model in the set of RL models, defined for (i) the composition selection, and (ii) each of the one or more sequential manufacturing process steps present in each of the one or more manufacturing process routes, in a reverse sequential order, by utilizing the training environment of the associated RL model and each training dataset present in the plurality of training data sets, to obtain the set of trained RL models

308e

FIG. 7

Start → Identify working range of all reward components → Normalize the reward components → Assign relative weights for various reward components within each process step

Assign relative weights for various reward components within each process step → Assign relative weights for final properties → Assign relative weights between global and local rewards → Assign constraint penalty factors for all constraint rewards → Stop

FIG. 8

FIG. 9

FIG. 10

Iterate(m)

Generate materials and process designs for each process route

Add generated designs as experience to each RL model memory

Train and update the RL models with additional experiences

Gather design requirements

Load trained RL models for each process route

Generated material designs
( m+n sets of )
Material and process design with route 1
Material and process design with route 2
.
.
Material and process design with route k

Generate materials designs for each process route with small random noise added to each model prediction

Iterate(n)

FIG. 11

Route 1

M-4
Rough
Rolling

M-1
Composition
generator

M-2
Casting

M-3
Reheating

M-5
Finish
Rolling

M-6
ROT +
coiling

Route 2

FIG. 12

| Requirements | | | YS | 245 MPa | UTS | 450 MPa | Elongation | 0.17 | Sheet Thickness | 7 mm |
|---|---|---|---|---|---|---|---|---|---|---|

| RS | Composition (wt%) | | | | | Casting | | Reheating | | Rough Rolling | | | Finish Rolling | | | ROT | Properties | | Sustainability | | |
|---|---|---|---|---|---|---|---|---|---|---|---|---|---|---|---|---|---|---|---|---|---|
| | C | Mn | Si | Cr | Ni | Route | CR | t | T | N | v | r | N | v | L | CR | W | Cor | E | Cost | CF |
| Y | 0.15 | 0.5 | 0.04 | 0.075 | 0 | CC | 5 | 1800 | 1100 | 4 | 1 | 90 | 5 | 0.59 | 82.9 | 10.9 | H | L | L | L | L |
| Y | 0.2 | 0.5 | 0.04 | 0.45 | 0 | CC | 5 | 1800 | 1100 | 4 | 1.26 | 90 | 5 | 0.8 | 150 | 7.9 | M | H | H | M | H |
| Y | 0.2 | 0.55 | 0.04 | 0.5 | 0 | TSC | 10 | 600 | 1000 | - | - | - | 6 | 0.25 | 114.7 | 4.5 | M | H | H | M | H |
| Y | 0.2 | 1.5 | 0.04 | 0.5 | 0 | TSC | 10 | 1216 | 1000 | - | - | - | 8 | 0.31 | 70.2 | 8.9 | L | H | H | H | H |

| RS | Requirement satisfied | CR | Coolig Rate (C/s) | v | Slab velocity (m/s) | H | High |
|---|---|---|---|---|---|---|---|
| Y | Yes | t | Time (Sec) | r | % Reduction | M | Medium |
| CC | Conventional Casting | T | Temperature (C) | L | Table length (m) | L | Low |
| TSC | Thin-Slab Casting | N | Number of roll passes | W | Weldability | E | Energy |
| | | | | | | CF | Carbon Footprint |

FIG. 13

FIG. 14

## EUROPEAN SEARCH REPORT

**Application Number**

EP 25 16 1977

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PENG GONGZHUANG ET AL: "A collaborative design platform for new alloy material development", ADVANCED ENGINEERING INFORMATICS, ELSEVIER, AMSTERDAM, NL, vol. 51, 10 December 2021 (2021-12-10), XP086982565, ISSN: 1474-0346, DOI: 10.1016/J.AEI.2021.101488 [retrieved on 2021-12-10] * abstract * * section 2, par. 1, section 3, par. 1, section 4.1, par. 1, section 4.3, par. 1 eq. 9; figures 3, 9, 11; tables 1, 4 * | 1-12 | INV. G06F30/27 G06N20/00 |
| A | ELTON PAN ET AL: "Deep Reinforcement Learning for Inverse Inorganic Materials Design", ARXIV.ORG, CORNELL UNIVERSITY LIBRARY, 201 OLIN LIBRARY CORNELL UNIVERSITY ITHACA, NY 14853, 21 October 2022 (2022-10-21), XP091350367, * abstract * * section 2, section 3.2, section A.2, A.3; figures 1, 2; table 1 * | 1-12 | |
| A | US 2022/261510 A1 (TAKEMOTO SHIMPEI [JP] ET AL) 18 August 2022 (2022-08-18) * abstract * * [0006]-[0018], [0107], [0152]-[0161]; figures 2, 12-14, 19 * | 1-12 | TECHNICAL FIELDS SEARCHED (IPC) G06F G06N |

The present search report has been drawn up for all claims

| Place of search | Date of completion of the search | Examiner |
|---|---|---|
| Munich | 2 July 2025 | Brandiska, Pavlina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 614 382 A1**

## ANNEX TO THE EUROPEAN SEARCH REPORT
## ON EUROPEAN PATENT APPLICATION NO.

EP 25 16 1977

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2025

| Patent document cited in search report | Publication date | Patent family member(s) | Publication date |
|---|---|---|---|
| US 2022261510 A1 | 18-08-2022 | CN 114144788 A | 04-03-2022 |
| | | EP 4006764 A1 | 01-06-2022 |
| | | JP 6950119 B2 | 13-10-2021 |
| | | JP WO2021015134 A1 | 18-11-2021 |
| | | US 2022261510 A1 | 18-08-2022 |
| | | WO 2021015134 A1 | 28-01-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421016615